# EUROPÄISCHE PATENTSCHRIFT

(11) **EP 2 224 939 B1**
(45) Veröffentlichungstag und Bekanntmachung des Hinweises auf die Patenterteilung: **18.03.2015**
(21) Anmeldenummer: 08865898.4
(22) Anmeldetag: 19.12.2008
(51) Int. Cl.: A61K 36/738, A61K 38/39, A61K 31/7008, A23L 1/30, A61P 19/02

(54) **ZUBEREITUNGEN MIT HAGEBUTTENEXTRAKTEN SOWIE VERFAHREN ZUR HERSTELLUNG VON HAGEBUTTENEXTRAKTEN**
PREPARATIONS WITH ROSEHIP EXTRACTS, AND METHOD OF PRODUCING ROSEHIP EXTRACTS
PRÉPARATION AVEC DES EXTRAITS DE CYNORHODON, ET PROCÉDÉ DE FABRICATION D'EXTRAITS DE CYNORHODON

(30) Priorität: 21.12.2007 EP 07123943
(43) Veröffentlichungstag der Anmeldung: 08.09.2010
(73) Patentinhaber: Finzelberg GmbH & Co. KG, 56626 Andernach (DE)
(72) Erfinder: WALBROEL, Bernd, 53639 Königswinter (DE); FEISTEL, Björn, 56626 Andernach (DE); PISCHEL, Ivo, 53547 Rossbach (DE)
(74) Vertreter: Von Kreisler Selting Werner - Partnerschaft von Patentanwälten und Rechtsanwälten mbB
(86) Internationale Anmeldenummer: PCT/EP2008/068081
(87) Internationale Veröffentlichungsnummer: WO 2009/080778

(56) Entgegenhaltungen:
- WO-A1-92/15314
- WO-A1-99/53934
- WO-A1-2008/003314
- WO-A2-03/043613
- WO-A2-2008/006589
- DE-U1-202005 017 053
- FR-A1- 2 897 238
- US-A- 4 004 976
- GAO X ET AL: "Evaluation of antioxidant activities of rosehip ethanol extracts in different test systems" JOURNAL OF THE SCIENCE OF FOOD AND AGRICULTURE, Bd. 80, Nr. 14, 1. November 2000 (2000-11-01), Seiten 2021-2027, XP003002393 WILEY & SONS, CHICHESTER, GB ISSN: 0022-5142
- KHARAZMI A ET AL: "Rose hip inhibits chemotaxis and chemiluminescence of human peripheral blood neutrophils in vitro and reduces certain infalmmatory parameters in vivo" INFLAMMOPHARMACOLOGY, Bd. 7, Nr. 4, 1. Januar 1999 (1999-01-01), Seiten 377-386, XP002237424 KLUWER ACADEMIC PUBLISHERS, DORDRECHT, NL ISSN: 0925-4692
- DATABASE CA CHEMICAL ABSTRACTS SERVICE, COLUMBUS, OHIO, US; 1963, SHMELEVA ET AL.: "Effect of vitamins C+P complex on lactic acid streptococci and the activity of its bacteriophage" XP002490525 Database accession no. 59-14326e-f
- DATABASE CA CHEMICAL ABSTRACTS SERVICE, COLUMBUS, OHIO, US; 1957, AL'TER: "Use of the enzyme pectinase in the preparation of cholosas" XP002490526 Database accession no. 52-47113
- DATABASE CA CHEMICAL ABSTRACTS SERVICE, COLUMBUS, OHIO, US; 1949, NOVOTEL'NOV: "Enzymic preparation of vitamin C concentrate enriched with vitamin P" XP002490527 Database accession no. 44-5890
- ANONYMOUS: "Joint Flex 1000 with Chondroitin & Rosehip" INTERNET ARTICLE, [Online] 2007, XP002543526 Gefunden im Internet: URL:http://www.auravita.com/products/aura/ HEPE10452.asp> [gefunden am 2009-08-27]
- CALLARD: "I feel it in my fingers..." NATURAL PRODUCTS, [Online] 20. Juli 2007 (2007-07-20), XP002543525 Gefunden im Internet: URL:http://www.naturalproductsonline.co.uk /home.asp?ItemID=3002&pcid=87&cid=88&archi ve=yes> [gefunden am 2009-08-27]
- ANONYMOUS: "Joint and Bone Combo" INTERNET ARTICLE, [Online] 10. November 2007 (2007-11-10), XP002542992 Gefunden im Internet: URL:http://jointandbonecombo.wordpress.com /> [gefunden am 2009-08-21]
- DATABASE WPI Week 200429 Thomson Scientific, London, GB; AN 2004-310679 XP002505223 & JP 2004 113141 A (DARWINS KK) 15. April 2004 (2004-04-15)
- DATABASE CA CHEMICAL ABSTRACTS SERVICE, COLUMBUS, OHIO, US; 1993, OSZMIANSKI ET AL.: "Possible use of enzymatic preparations in the production of cloudy juices of high vitamin content from fruits of the rose Rosa rugosa; rosehip pectin degradation and juice preparation with high ascorbic a cid content" XP002490528 Database accession no. 1993-11708
- DELIORMAN ORHAN DIDEM ET AL: "In vivo anti-inflammatory and antinociceptive activity of the crude extract and fractions from Rosa canina L. fruits." JOURNAL OF ETHNOPHARMACOLOGY, Bd. 112, Nr. 2, 13. Juni 2007 (2007-06-13), Seiten 394-400, XP002490522 ISSN: 0378-8741
- CHRUBASIK C ET AL: "The evidence for clinical efficacy of rose hip and seed: a systematic review." PHYTOTHERAPY RESEARCH : PTR JAN 2006, Bd. 20, Nr. 1, Januar 2006 (2006-01), Seiten 1-3, XP002490523 ISSN: 0951-418X
- DATABASE WPI Week 200770 Thomson Scientific, London, GB; AN 2007-740658 XP002490529 & CN 1 928 098 A (TONG Z) 14. März 2007 (2007-03-14)
- MUNOZ O ET AL: "Effects of enzymatic treatment on anthocyanic pigments from grapes skin from chilean wine" FOOD CHEMISTRY, Bd. 87, Nr. 4, Oktober 2004 (2004-10), Seiten 487-490, XP002542993 ISSN: 0308-8146
- LEE Y N ET AL: "PURIFICATION AND CONCENTRATION OF BETALAINES BY ULTRA FILTRATION AND REVERSE OSMOSIS" JOURNAL OF FOOD SCIENCE, Bd. 47, Nr. 2, 1. Januar 1982 (1982-01-01), Seiten 465-471,475, XP008094339 INSTITUTE OF FOOD TECHNOLOGISTS, CHICAGO, IL, US ISSN: 0022-1147
- CHRUBASIK COSIMA ET AL: "A systematic review on the Rosa canina effect and efficacy profiles." PHYTOTHERAPY RESEARCH : PTR JUN 2008, Bd. 22, Nr. 6, Juni 2008 (2008-06), Seiten 725-733, XP002490524 ISSN: 1099-1573
- [Online] 02 März 2004, Gefunden im Internet: <URL:http://www.martin-bauer-group.com/de/n ewspr/news/article/nutrifin-konzepte-fuer-d ie-gesundheit.html> [gefunden am 2012-03-23] 'Extrakte gegen Erkältung, Gelenkschmerzen und Erektionsstörungen', [Online] 24 September 2010, Gefunden im Internet: <URL:http://www.the-nature-network.com/de/n ewspr/news/article/extrakte-gegen-erkaeltun g-gelenkschmerzen-und-erektionsstoerungen.h tml> [gefunden am 2012-03-26]
- MOSKOWITZ R W: "ROLE OF COLLAGEN HYDROLYSATE IN BONE AND JOINT DISEASE", SEMINARS IN ARTHRITIS AND RHEUMATISM, ELSEVIER, AMSTERDAM, NL, vol. 30, no. 2, 1 October 2000 (2000-10-01), pages 87-99, XP009021767, ISSN: 0049-0172, DOI: 10.1053/SARH.2000.9622
- SKELETIN - DOKUMENTACJA DO PRODUKTU (ENERGY) PAGES 1-15; ADDITIONALLY 3 PAGES OF SELECTED CHAPTERS TRANSLATED INTO ENGLISH
- GOOGLE SEARCH RESULTS FOR THE EXPRESSION "SKELETIN" WITHIN THE TIME PERIOD OF 01.01.2000-12.12.2007
- PRODUCT INFORMATION REGARDING "ARTREVIT" IN POLISH (1 PAGE), AND A TRANSLATION INTO ENGLISH
- GOOGLE SEARCH RESULTS FOR THE EXPRESSION "ARTREVIT" WITHIN THE TIME PERIOD OF 01.01.2000-12.12.2007

## Beschreibung

Die vorliegende Erfindung betrifft Zusammensetzungen enthaltend einen entzündungshemmenden Pflanzenextrakt aus Hagebutte zusammen mit Kollagenhydrolysaten. sowie ein Verfahren zur Herstellung derselben.

### Hintergrund der Erfindung

Immer mehr Menschen sind von Arthrose (Gelenkverschleiß, Osteoarthritis, Arthrosis deformans) betroffen. Diese Erkrankung gehört zum rheumatischen Formenkreis und geht in vielen Fällen und vor allem in akuten Phasen mit schmerzhaften Entzündungen einher. Ursache für die Schmerzen ist eine Zerstörung des Gelenkknorpels. Verschiedene Faktoren können zu einem Knorpelschaden führen. Neben den Schäden in Folge von Unfällen, Überlastungen oder angeborenen Fehlformen der Gelenke gehören Stoffwechselstörungen, Bewegungsarmut und auch Ernährungsfehler zu den wichtigsten Ursachen.

Ausgangspunkt jeder Arthrose ist ein Schaden im Knorpelüberzug, der so genannte "Knorpelschaden". Oft ist dieser Schaden zunächst nur auf eine kleine Fläche von wenigen Quadratzentimetern begrenzt. Außerdem ist er noch oberflächlich. Kurz darauf treten im Röntgenbild erste Verdichtungen des Knochens auf. Es handelt sich hierbei immer um Knochenbezirke, die direkt unter dem erkrankten Knorpel liegen. Diese zusätzlichen Veränderungen am Knochen sind ein entscheidendes Zeichen für das Frühstadium der Arthrose. Ohne diese Knochenveränderungen liegt nur ein "Knorpelschaden" vor, nicht aber eine "Arthrose". Arthrose bedeutet deshalb immer Knorpelschaden mit Knochenveränderungen.

Für die betroffenen Personen bedeutet die Arthrose eine schmerzhafte, chronische Krankheit, die sie bei fast allen Tätigkeiten beeinträchtigt. Schmerzen, Entzündungsschübe, Verdickung und Verformung sowie Versteifungen der Gelenke sind die Folgen. Für jedes Gelenk und jedes Stadium können Ausmaß und Ausprägung dieser Vorgänge aber sehr verschieden sein. Die Bewegungsfreiheit ist stark eingeschränkt.

Bei ihrer Suche nach Linderung greifen viele Patienten zu frei verkäuflichen oder rezeptpflichtigen Medikamenten, unterziehen sich langwierigen Physiotherapien oder lassen sich sogar operieren. Jedoch bringen ihnen all diese Behandlungen oft nur eine kurzfristige Linderung der Schmerzen. In den meisten Fällen schreitet die Zerstörung des Knorpels unaufhaltsam fort, mit der Folge, dass die Schmerzen größer und die Bewegungseinschränkungen gravierender werden.

Eine Entzündung (*lateinisch Inflammatio*) ist eine charakteristische Antwort von biologischem Gewebe auf einen äußeren oder innerlich ausgelösten Reiz mit der Funktion, den Schädigungsreiz zu beseitigen oder zu reparieren. Eine Entzündung kann in einem umschriebenen Gebiet oder als systemische Entzündungsreaktion vorliegen. Im vorliegenden Beispiel stellt die Arthritis einen großen schmerzbestimmenden Faktor der Arthrose dar. Die fünf Entzündungszeichen (Rötung, Überwärmung, Schwellung, Schmerz und eingeschränkte Funktion) lassen sich bei der Arthritis in den meisten Fällen sämtlich innerhalb des chronischen Verlaufs feststellen. So stellen die Rötung und die Überwärmung oft ein, wenn auch kurzes, Warnzeichen eines beginnenden Entzündungsschubes dar, an den sich die Schmelzphase bald anschließt. Die Schwellung wird häufig bei den oft schon verbreiterten Gelenken kaum mehr wahrgenommen. Die eingeschränkte Funktion ist dann als Folge der Schmerzen anzusehen, in ausgeprägten Formen als Folge von Fehlstellungen.

Es gibt somit zwei Wege durch Knorpelschäden-bedingte Entzündungen zu bekämpfen und damit auch die Schmerzsituation und Gelenksteife für die Patienten zu verbessern:
Der erste Weg liegt in der unterstützenden Selbstheilung des Körpers durch knorpelprotektive Substanzen. Im Handel sind hierzu Nahrungsergänzungsmittel mit Kollagenhydrolysat für gesündere und leistungsfähigere Gelenke, welche das Gelenkkollagen stärken bzw. dessen Neubildung unterstützten. In Orthopädische Praxis (2005,10, 41: 565-568*)* beschreibt Dr. S. Oesser den Einfluss der Kollagenfragmente auf die Neusynthese und Degradation extrazellulärer Knorpelmatrix. Das in dieser Veröffentlichung und in den Beispielen dieser Anmeldung verwendete Kollagenhydrolysat stammt aus Kollagen des Typs 1. Dieses wirkt stimulierend auf die Bildung des Kollagens vom Typ 2, sowie die pericelluläre Proteoglykan- Biosynthese. Kollagen vom Typ 2 ist mit einem Anteil von ca. 70% der mengenmäßig wichtigste Baustein (des Gelenkknorpels) und sorgt hier für Elastizität und Zugfestigkeit. Eine Studie von Dr. Roland W. Moskowitz in Semin Arthritis Rheum (2000, 30: 87-99*)* kommt zu dem Schluss, dass eine Tagesdosis von 10g Kollagenhydrolysat zu einer deutlichen Schmerzreduktion arthritischer Patienten führt.

Weiterhin sind Präparate mit Glucosaminsulfat (Tagesdosis 0,75g - 1,5g) und / oder Chondroitinsulfat (Tagesdosis 0,4g - 0,8g) im Handel, welche ebenfalls eine knorpelprotektive Wirkung für sich in Anspruch nehmen. Eine Veröffentlichung von A.A.Brief, im J. Am. Acad. Orthop.Surg. (2001, 9: 71-78*)* belegt eine entsprechende Wirkung.

Der zweite Weg ist die Unterdrückung der Entzündung bzw. die Reduzierung entzündungsvermittelnder Mediatoren. Diese Mediatoren, z.B. Zytokine, sind für eine zu heftige Abwehrreaktion zuständig.

Die natürliche Antwort des Körpers zur Bekämpfung von Entzündungen ist die Ausschüttung von Corticoiden. Es lag also nahe, Cortisol-Abkömmlinge zu synthetisieren und diese als antiinflammatorische Medikamente in den Handel zu bringen. Prednisolon und Dexamethason sind noch heute als stark wirksame Arzneimittel die letzte Rettung. Jedoch führt die Langzeitbehandlung zu starken Nebenwirkungen, wie Striae, Muskelathrophie, Blutbildveränderungen oder Diabetes mellitus Typ 2.

Mittel der Wahl zur Langzeittherapie sind heute die nichtsteroidalen Antirheumatika (NSAR) und COX-2-Inhibitoren. Doch auch Diclofenac, Ibuprofen, Indometacin und Oxicame haben ein Nebenwirkungspotential, welches von Magen-Darm-Beschwerden über Magengeschwüre bis zu Leber- oder Nierenschäden reicht.

Aus dem Bereich der Naturheilkunde sind in vielen Kulturen schon seit vielen hundert Jahren Heilkräuter verwendet worden, welche sich üblicherweise durch eine gute Verträglichkeit auszeichnen. Zu den klassisch anerkannten Phytopharmaka zählt u.a. die Teufelskralle. Während das wirksame Prinzip beispielsweise der Weidenrinde mit Salicin aufgeklärt zu sein scheint, werden andere Pflanzen noch stark beforscht.

Es hat sich nun erstaunlicherweise die Hagebutte als klassische Nahrungspflanze als potentes Antiphlogistikum herausgestellt. Die Hagebutte ist eine Sammelfrucht, die viele kleine Nüsse enthält. Das Fruchtfleisch der im Spätherbst geernteten Früchte entsteht aus dem fleischigen Blütenboden, ist süßsauer und reich an Vitaminen, insbesondere Vitamin C (Ascorbinsäure), aber auch Vitamin A, B1 und B2. Traditionell werden Hagebutten zur Vitamin-C-Substitution bei Erkältungskrankheiten und grippalen Infekten verabreicht. Vitamin C spielt ebenfalls bei der Neubildung von Kollagen im Gelenkknorpel eine wichtige Rolle und ist für den Erhalt von Knochen und Stützgewebe notwendig. Das Patent US 6,024,960 beschreibt den Zusammenhang eines hohen Vitamin C-Gehaltes mit einer antiinflammatorischen Wirkung von Zubereitungen aus Hagebutten. In der Patentschrift US 6,485,752 B1 wird die Kombination eines Hagebuttenkonzentrates mit Fischöl geschützt, welcher auf hohe Vitamin C-Gehalte im Extrakt und auf ungesättigte Fettsäuren im Fischöl abzielt.

Berichten zufolge kann eine Verbesserung der Beweglichkeit und des Wohlbefindens durch Einnahme von Hagebuttenpulver erreicht werden. Eine Forschergruppe um Prof. Dr. Kharazmi an der Universität Kopenhagen studierte 2004 die Effekte eines Hagebutten-Pulvers bei Gelenks-Arthrosen, und isolierte mittels eines "komplizierten Fraktionierungsverfahrens" einen Galaktolipid-Anteil. Das Galaktolipid "GOPO^{®}" wurde in Verbindung mit dem Herstellungsverfahren für Hagebutten-Pulver patentiert (EP 1 071 439). Es konnte in-vitro nachgewiesen werden, dass dieses Galaktolipid die Migration polymorphkerniger Leukozyten hemmt und in-vivo die Serumkonzentrationen an C-reaktivem Protein (CRP) senkt. In einer klinischen Studie sank der CRP-Wert bereits nach 10 Tagen um durchschnittlich 39%. Eine placebokontrollierte cross-over Studie wurde mit 112 Arthrose-Patienten durchgeführt, dabei wurde die tägliche Gabe von 5 Gramm Hagebutten-Pulver (LitoZin^{®}) untersucht. Die Studie zeigte eine signifikante Linderung der Morgensteifigkeit bei 66% der Studienteilnehmer nach dreimonatiger Einnahme des Hagebuttenpulvers. Außerdem konnte der Konsum an Schmerzmitteln wie Opioiden, Tramadol, Paracetamol oder NSAR bei der Ernährungstherapie mit Hagebuttenpulver um rund die Hälfte vermindert werden. Das im Produkt LitoZin^{®} verwendete Hagebuttenpulver ist auf eine mit 175 ppm enthaltene Substanz standardisiert und bei optischer Betrachtung als inhomogen zu beschreiben. Dies drückt sich in schwankenden Korngrößenverteilungen über mehrere Chargen aus (Grobpartikel von 20% zwischen 0,5-0,7mm bis hin zu Feinstanteilen von 40% zwischen 0.05-0,2 mm). Ein systematischer Überblick von C.Chrubasik et al. in Phytotherapy Research (2006:20:1-3*)* fasst die klinischen Daten von Hagebuttenpulvern zusammen.

JP20020281736 offenbart eine entzündungshemmende Zusammensetzung, die Hagebutte in Kombination mit einer nicht näher beschriebenen Kollagenkomponente enthält. Die Zusammensetzung wird als Nahrungsergänzung zur Prävention von Gelenkbeschwerden eingesetzt.

Die eingesetzten Hagebutten-Pulver sind schlecht charakterisiert bzw. standardisiert. Die einzunehmenden Mengen von bis zu 10g sind aufgrund der geringen Konzentrationen der Wirkstoffe sehr groß. Es besteht daher weiterhin ein Bedarf nach charakterisierten und / oder standardisierten- Hagebuttenprodukten, die eine hohe Wirksamkeit pro Gewichtseinheit aufweisen.

Die Aufgabe der vorliegenden Erfindung besteht in der Bereitstellung eines gutverträglichen, entzündungshemmenden Mittels, insbesondere zur Behandlung von Gelenkbeschwerden und -erkrankungen.

Gelöst wird die Aufgabe durch eine Zusammensetzung enthaltend einen entzündungshemmenden Pflanzenextrakt aus Hagebutte zusammen mit Kollagenhydrolysat.

### Detaillierte Beschreibung der Erfindung

Ziel der Erfindung war die Entwicklung eines Extraktes aus einer wenig genutzten Nahrungspflanze für die Bekämpfung oder Vorbeugung von Entzündungsreaktionen. Bevorzugt handelt es sich um eine Extraktzubereitung, die während oder nach dem Trocknungsprozess mit knorpelprotektiven Trocknungshilfsstoffen kombiniert wird. Außerdem sollte ein Extrakt entwickelt werden, welcher ohne übermäßig temperaturlabile (Galaktolipide), nebenwirkungsrelevante (Salizylate), oxidationsempfindliche (Ascorbinsäure) oder rein lipophile (Triterpensäuren) Komponenten wirksam ist.

Die Wirkungsweise von Hagebuttenextrakten lässt sich auf folgende Weise charakterisieren: Durch Zytokine "angelockte" Leukozyten sind am entzündlichen Prozess in den Gelenken beteiligt. Überraschend konnte festgestellt werden, dass der Hagebuttenextrakt die Ausschüttung der Zytokine vermindert, so dass weniger Leukozyten in das Entzündungsgebiet einwandern und so das Knorpelgewebe weiter schädigen.

Das Knorpelgewebe wird aber auch durch die Bildung freier Radikale aus dem Entzündungsprozess geschädigt. Überraschend konnte festgestellt werden, dass der Hagebuttenextrakt unabhängig von seinem natürlichen Ascorbinsäure-Gehalt auch diese Bildung freier Radikale reduziert. So schwächt der Hagebuttenextrakt die Entzündungsreaktion in den Gelenken ab oder unterdrückt sie sogar ganz. Dadurch werden Schädigung und Zerstörung des Knorpels gestoppt und sowohl die Schmerzen gelindert, als auch die Beweglichkeit verbessert.

Die Erfindung beschreibt die Herstellung und Verwendung eines Extraktes aus Hagebutten sowie dessen Kombination mit knorpelprotektiven Substanzen wie Kollagenhydrolysat, Glucosamin und/oder Chondroitinsulfat zur Gesunderhaltung und/oder Reduzierung von Symptomen bei Beschwerden des rheumatischen Formenkreises, speziell bei chronischen Gelenksentzündungen, wie der rheumatoiden Arthritis, sowie ähnlichen Beschwerden. Eine besonders bevorzugte Ausführungsform besteht in der Verwendung einer knorpelprotektiven Substanz als unmittelbarer Trocknungshilfsstoff für den Hagebuttenextrakt.

In einer bevorzugten Ausführungsform wird ein Hagebuttenextrakt eingesetzt, der durch ein Verfahren zur Herstellung eines Trockenextraktes aus Hagebutten mit folgenden Schritten:
a) Extraktion von Hagebutten (Rosa canina) mit Wasser oder einer Mischung von Wasser und bis zu 50 Gew.-% Ethanol um einen Einfachextrakt zu erhalten
b) Aufreinigung des erhaltenen Einfachextraktes durch mindestens einen der Schritte
   b1) enzymatische Fermentierung
   b2) Membranfiltration
c) Trocknung des Extraktes
erhältlich ist.

Es können Hagebuttenschalen oder Hagebuttenschalen und -kerne eingesetzt werden.

Bevorzugt wird als Droge Fructus Cynosbatum DAB oder Cynobastum sine semine eingesetzt.

Überraschend ist es gelungen, einen Extrakt zu entwickeln, welcher in einer Ausführungsform unter Zuhilfenahme eines Enzyms vorbehandelt wird. Eine Anreicherung des aktiven Prinzips war in einer anderen Ausführungsform darüber hinaus über das Verfahren der selektiven Membranfiltration zu erzielen. Enzymbehandlung und Membranfiltration können auch kombiniert werden.

Eine Unterdrucktrocknung im Temperaturbereich bis 80°C wirkte sich überraschenderweise nicht nachteilig auf die Aktivität aus.

Für die enzymatische Behandlung haben sich insbesondere hydrolytische Enzyme, insbesondere Glycosidasen als wirksam erwiesen. Andere geeignete Enzyme sind Cellulasen wie beispielsweise Hemicellulasen, insbesondere Xylanase. Besonders bevorzugt eingesetzte Enzyme sind Pektinasen.

Membranfiltration ist ein Filtrationsverfahren, bei dem durch eine Membran filtriert wird und auch kleinste Partikel entfernt werden können. Bevorzugt wird die Membranfiltration als Ultrafiltration durchgeführt, wobei Substanzen auf Grund ihrer Molekülgröße entfernt werden können und das Permeat weiter verwendet wird. Geeignete Ausschlussgrößen für die Membranen lagen bei 1 kDa bis 500 kDa, mehr bevorzugt bei 10 kDa bis 300 kDa, speziell bei 100 kDa.

Synergistische Effekte in der Schmerzreduktion und der Dauer der Symptomausprägung konnten durch die Unterstützung der Selbstheilungskräfte der Knorpelregeneration beobachtet werden. Verwendung findet ein Extrakt aus Hagebutten aus der Spezies Rosa canina. Der bevorzugte Extrakt enthält keine nachweisbaren Spuren an Galaktolipid "GOPO^{®}", sowie nur einen rudimentären Gehalt an Salicylaten. Der Gehalt an lipophilen pentacyclischen Triterpensäuren ist ebenfalls minimal.

Der Nachweis der dosisabhängigen antiinflammatorischen Wirkung wurde in einer Studie erbracht, in welcher die Inhibition einer durch Lipopolysaccharide (LPS) induzierten Ausschüttung von Entzündungsmediatoren an humanen Monozyten gemessen wurde. Diese waren im Einzelnen die Cytokine Interleukin-1ß (IL-1ß), Interleukin-6 (IL-6), Prostaglandin E₂ (PGE₂), sowie der Tumornekrosefaktor-alpha (TNF-α). PGE₂ ist eines der "Hauptprostaglandine", welches in das Entzündungsgeschehen involviert ist. Es erhöht die Gefäßpermeabilität (Gewebeschwellung), ist an der Entstehung der Rötung beteiligt und verstärkt den Schmerz (welcher durch andere Entzündungsstoffe wie Bradykinin oder Histamin hervorgerufen wird), indem es nozizeptive Nervenendigungen sensibilisiert.

In einer Studie konnte der Beweis erbracht werden, dass die erfindungsgemäßen GOPO^{®}-freien, weitestgehend Salicylat-freien und Pseudosaponinminimierten wässrigen Extrakte eine sehr gute entzündungshemmende Wirkung hatten.

Wurde nun durch zugesetzte Knorpelprotektiva wie Kollagenhydrolysat, Glücosamin oder Chondroitinsulfat die Neubildung von Gelenkkollagen gefördert, so konnte auf diese Weise die Selbstheilung von Knorpelschäden unterstützt werden; Spätfolgen wie die Arthrose könnten somit reduziert werden.

In einer Ausführungsform konnte ein rein wässriger, aber dennoch aktiver Hagebuttenextrakt entwickelt werden, der nachweislich ohne hitze- und lichtempfindliche Verbindungen der Stoffgruppe der Galaktolipide auskommt, so gut wie keine nebenwirkungsrelevanten Salizylate enthält, sowie nicht auf die Verbindungsklasse der lipophilen Pseudosaponine angewiesen ist.

Diese antiinflammatorischen Effekte werden erfindungsgemäß mit der knorpelschützenden und regenerationsfördernden Komponente des Kollagenhydrolysates kombiniert. Eine weitere bevorzugte Ausführungsform ist die Kombination des Extraktes mit dem osteoarthritsich wirksamen Kollagenhydrolysat sowie einer Ca-Supplementation in Form von Calciumpyruvat.

Die Anwendung als Nahrungsergänzungsmittel und in bilanzierten Diäten ist möglich; die Zielgruppe sind Personen mit chronischen Gelenksentzündungen, zur Verminderung der verwendeten NSAR-Mengen, sowie Leistungssportler, zur beschleunigten Regeneration in Rehabilitationsphasen nach Gelenkstauchungen und Bandscheibenüberlastungen.

Ein weiterer Gegenstand der Erfindung ist eine Zusammensetzung enthaltend einen entzündungshemmend-wirkenden Pflanzenextrakt aus Hagebutte zusammen mit Kollagenhydrolysaten. Weitere entzündungshemmende Pflanzenextrakte sind Extrakte von Ackerschachtelhalm, Afrikanische Pflaume, Amarant, Angelika, Arnika, Beinwell, Basilikum, Bärlapp, Bärlauch, Ballonblume, Borretsch, Brennessel, Brombeere, Brokkoli, Buchweizen, Butterblume, Capsicum, Curcuma, Cucurbita, Duftveilchen, Ehrenpreis, Eisenkraut, Enzian, Estragon, Eykalyptus, Galgant, Gewürznelke, Giersch, Goldrute, Holunder, Ingwer, Kamille, Kapuzinerkresse, Kardamom, Kirsche, Koriander, Lakritz, Lemongras, Lindenblüte, Lorbeer, Mangosteen, Mädesüß, Majoran, Mariendistel, Meerrettich, Melisse, Minze, Mutterkraut, Olive, Perilla, Pfeffer, Ringelblume, Rosmarin, Salbei, Schafgarbe, Schleifenblume, Schlüsselblume, Sellerie, Senf, Silberweide, Thymian, Veilchen, Vogelmiere, Vogelknöterich, Waldmeister, Weidenrinde, Wermut, Ysop, Zimt, Zistrose, Zwiebel und Mischungen davon.

Entsprechende Extrakte eignen sich für Arznei-, Nährungs- oder Ergänzungsmittel zur Prävention oder Reduzierung von Symptomen bei chronischen Gelenkentzündung, rheumatoider Arthritis, Arthrose, Erkrankungen des rheumatischen Formkreises, Spondylitis, Osteoarthritis, Fibromyalgie oder zur Unterstützung der Rehabilitation nach Gelenkverstauchung oder Bandscheibenstauchungen.

### Beispiele

### Beispiel 1: Einfluss des Auszugsmittels auf das antiinflammatorische Potential von Cynosbati-Extrakten

Aus je 1 kg Hagebuttenschalen (Rosa canina) wurde zweimal mit je 6 Litern Lösemittel bei 50°C für die Dauer von 6h extrahiert. Die Auszüge wurden über Nacht absetzen gelassen, vereinigt und am nächsten Morgen klarfiltriert. Anschließend wurde zum Spissum lösemittelfrei einrotiert und mit 50% Maltodextrin bei 50°C im Vakuum getrocknet. Die so erhaltenen Extrakte wurden auf Ihr Potential zur Hemmung typischer Entzündungsparameter wie TNFalpha oder typischer Schmerzparameter wie PGE2 an humanen Monozyten geprüft.

| Auszugsmittel | PGE2 IC50 [µg/ml] | TNF-alpha IC20 [µg/ml] |
|---|---|---|
| 70% EtOH V/V | 450 | 200 |
| 45% EtOH V/V | 200 | 100 |
| 20% EtOH V/V | 300 | 325 |
| Wasser | 450 | 100 |
| Wasser - PEG 300 | 450 | 200 |
| Wasser - Glycerol | 450 | 300 |
| Drogenpulver | >> 500 | 350 |

Das Cynosbati-Drogenpulver weist eine unspezifische, kaum messbare Inhibierung von PGE2 auf. Demgegenüber waren Extrakte, besonders wässrig oder wässrig-ethanolische Extrakte dosisabhängig messbar. Eine mittlere 50%ige Inhibition (IC50) war bereits ab 200 µg/mL (mind. 5fach verstärkte Wirkung) messbar.

Ferner zeigen die Extrakte bei der Inhibierung des Entzündungsparameters TNFalpha eine deutlich stärkere, ebenfalls dosisabhängige Wirkung. Da das Drogenpulver maximal eine 20%ige Inhibierung erreichte, wurde für den Vergleich der IC20-Wert herangezogen.

### Beispiel 2: Aufreinigung durch enzymatische Behandlung

Aus 1 kg Hagebuttenschalen wurde zweimal mit je 6 Litern Wasser bei 50°C extrahiert. Die Auszüge wurden über Nacht absetzen gelassen, vereinigt und am nächsten Morgen klarfiltriert. Nach Zusatz von 3g Ultrazym^{®} pro 2 kg Trockensubstanz wurde über 2 Tage bei Raumtemperatur fermentiert. Niederschläge wurden von der Lösung mittels Filtration abgetrennt. Der Überstand wurde anschließend zum Spissum einrotiert (native Extraktausbeute 29%) und mit 50% Maltodextrin bei 50°C im Vakuum getrocknet.

| | Wässriger Extrakt | Wässriger Extrakt nach Fermentation |
|---|---|---|
| PGE2 - IC50 [µg/ml] | 450 | 400 |
| TNF-a - IC20 [µg/ml] | 100 | 50 |

Der fermentierte Extrakt war gekennzeichnet durch einen Gehalt an 10,5% Polyphenole, hingegen war Ascorbinsäure nicht messbar (<0,04%). Der Gehalt an pentacyclischen Triterpensäuren war unterhalb der Nachweisgrenze (< 10 ppm) und es konnte keine durch Hydrolyse freisetzbare Linolensäure nachgewiesen werden (kein Galactolipid GoPo^{®}; <10 ppm). Das antiinflammatorische Potential des aufgereinigten Extraktes konnte bei TNF-alpha um 50% erhöht werden.

### Beispiel 3: Aufreinigung mit Membranfiltration

Der wässrige Spissum aus Beispiel 1 wurde auf einen TS von 20% mit Osmosewasser verdünnt und über eine Ultrafiltration Ausschlussgröße 100 kDa in zwei Fraktionen aufgetrennt. Anschließend wurde zum Spissum einrotiert und mit 50% Maltodextrin bei 50°C im Vakuum getrocknet.

| | Ausgangs-Extrakt | Permeat | Retentat |
|---|---|---|---|
| PGE2 - IC50 [µg/ml] | 450 | 350 | >> 500 |
| TNF-a - IC20 [µg/ml] | 100 | <50 | 300 |

Mit der erfindungsgemäßen Aufreinigung via Ultrafiltration konnte das antiinflammatorische Potential von TNFalpha um über 50% erhöht werden. Auch die PGE2-Aktivität konnte um über 20% gesteigert werden. Der Extrakt enthielt keine lipophilen Substanzen (z.B. Galactolipid GoPo^{®} <10 ppm). Das wirksame Prinzip, enthalten in Form wasserlösliche Verbindungen, konnte durch die selektive Auftrennung an einem definierten Membranfilter, weiter angereichert werden.

### Beispiel 4a: Darstellung eines Trockenextraktes unter Zuhilfenahme knorpelprotektiver Trockenhilfsstoffe

Ein Dickextrakt aus Hagebuttenschalen, gewonnen nach den Extraktionsbedingungen von Beispiel 1 mit dem Auszugsmittel Ethanol 30% V/V ergab eine native Extraktausbeute von 38%. Nach Entfernung des Lösemittels im Vakuum wurde die wässrige Extraktlösung einer enzymatischen Aufreinigung gemäß Beispiel 2 unterzogen. Es konnte eine native Extraktmenge von 31% erhalten werden: Diese Extraktlösung wurde auf einen TS von 30% mit Osmosewässer verdünnt und mit 50% Kollagenhydrolysat vom Typ 1 (Gelita Sol D) unter Rühren homogenisiert und sprühgetrocknet. Es resultierte ein rot-beiges Trockenpulver. Der Extrakt enthielt keine lipophilen Substanzen (z.B. Galactolipid GoPo^{®} <10 ppm), war komplett wasserlöslich mit einem angenehmen, beerenartigem Geschmack.

### Beispiel 4b: Darstellung eines Trockenextraktes unter Zuhilfenahme knorpelprotektiver Trockenhilfsstoffe

Ein Dickextrakt aus Hagebuttenschalen, gewonnen nach den Extraktionsbedingungen von Beispiel 1 mit dem Auszugsmittel Wasser, ergab eine native Extraktausbeute von 45%. Nach Entfernung des Lösemittels im Vakuum wurde die wässrige Extraktlösung einer enzymatischen Aufreinigung gemäß Beispiel 2 unterzogen. Es konnte eine native Extraktmenge von 38% erhalten werden. Diese Extraktlösung wurde auf einen TS von 30% mit Osmosewasser verdünnt und mit 30% Kollagenhydrolysat vom Typ 1 (Gelita Sol LDA) unter Rühren homogenisiert und vakuumgetrocknet. Es resultierte ein rot-beiges Trockenpulver. Der Trockenextrakt mit 4,8% Restfeuchte enthielt keine lipophilen Substanzen (z.B. Galactolipid GoPo^{®} <10 ppm; Gesamt-Fett 0,08%), war komplett wasserlöslich mit einem angenehmen, beerenartigem Geschmack. Der Ascorbinsäuregehalt lag bei 0,1%. Das Gesamteiweiß betrug nach Nährwertanalyse gemäß ASU, §64 LFGB, 31,8% (hiervon 5,1% Stickstoff). Der Kohlenhydratabteil lag bei 57,7g/100g, was in einem Brennwert von 1525 KJ/100g resultierte.

### Vergleichsbeispiel 5 : Extrakt von Thymian mit Kollagenhydrolysat

1 kg getrocknetes und geschnittenes Thymiankraut (Herba Thymii) wurden zweimal mit je 8 Litern gereinigtem Wasser bei 80°C für die Dauer von 8h erschöpfend extrahiert. Es wurden die Eluate über die Droge abfiltriert, vereinigt und final über einen Schichtenfilter klarfiltriert. Anschließend wurde zum Dickextrakt lösemittelfrei im Vakuum beigedampft, wobei der aetherische Ölanteil weitestgehend entfernt wurde. Anschließend würde die wässrige Extraktlösung einer Membranfiltration unterzogen.

Diese Extraktlösung wurde im Vakuum auf einen Trockensubstanzanteil von ca. 40% aufkonzentriert und anschließend einer Flüssig-Flüssig-Behandlung mit n-Heptan zur Entfernung sämtlicher lipophiler Substanzen wie Wachse, Harze oder aetherischer Öle unterzogen. Die verbliebene Wasserphase wurde von ca. 10% lipophiler Substanzen befreit. Der hiernach gewonnene Dickextrakt ergab eine native Extraktausbeute von 25 %.

Diese Extraktlösung, auf einen Trockensubstanzanteil (TS) von 32% eingestellt, wurde mit 20% Kollagenhydrolysat (Gelita Sol LDA) versetzt, unter Rühren homogenisiert und sprühgetrocknet.

Es resultierte ein braun-beiges Trockenpulver. Der Extrakt enthielt kein aetherisches Öl (z.B. Thymol < 10 ppm), ca. 3% Polyphenole (UV-VIS) und war komplett wasserlöslich.

### Vergleichsbeispiel 6 : Extrakte von Russischem Estragon mit Kollagenhydrolysat

1 kg getrocknetes und geschnittenes Estragonkraut (Herba Artemisia drancunculoides) wurden zweimal mit je 9 Litern gereinigtem Wasser bei 80°C für die Dauer von 6 h extrahiert. Die Auszüge wurden über die Droge abfiltiriert, vereinigt und final über einen Schichtenfilter klarfiltriert. Anschließend wurde zum Dickextrakt lösemittelfrei im Vakuum beigedampft, wobei der aetherische Ölanteil vollkommen entfernt wurde. Anschließend wurde die wässrige Extraktlösung einer Membranfiltration unterzogen.

Der hiernach gewonnene Dickextrakt ergab eine native Extraktausbeute von 33%.

Diese Extraktlösung wurde auf einen TS von 30% im Vakuum eingeent und mit 30% Kollagenhydrolysat (Gelita Sol LDA) unter Rühren homogenisiert und sprühgetrocknet.

Es resultierte ein braun-beiges Trockenpulver. Der Extrakt enthielt kein aetherisches Öl (z.B. Methyleugenol < 10 ppm), ca. 1% Flavonoide nach HPLC und war komplett wasserlöslich.

### Vergleichsbeispiel 7: Extrakt von Ingwer mit Kollagenhydrolysat und Glucosamin

1 kg getrocknete und geschnittene Ingwerwurzeln (Rhizoma Zingiberis officinalis) wurden zweimal mit je 12 Litern Ethanol bei 45°C für die Dauer von 4 h extrahiert. Die Auszüge wurden über die Droge abfiltiriert, vereinigt und auf einen Trockensubstanzanteil von ca. 20% schonend im Vakuum beigedampft. Die native Extraktausbeute betrug 10%.

Eine Mischung von 80% Polyvinylpyrrolidon (Kollidon 25) und 20% Kollagenhydrolysat (Gelita Sol LDA) wurden auf ebenfalls 20% Trockensubstanzanteil in Ethanol 50% gelöst.

Anschließend wurden beide Lösungen im Mengenverhältnis 1:4 eingewogen und unter ständigem Rühren portionsweise miteinander homogenisert. Das so entstehende Ingwer-Copräzipitat, die Einbindung der temperatur- und säureempfindlichen Ingwer-Scharfstoffe in die Matrix aus Kollidon und Kollagenhydrolysat, wurde im Vakuum vom Lösemittel befreit und zu einer Trockenextraktzubereitung getrocknet. 200 mg einer solchen Zubereitung werden mit 400 mg Glucosamin gemischt und über eine Zwangsvermahlung via 0,5 mm Sieb zu einem homogenen, frei fließenden Pulver verarbeitet.

### Vergleichsbeispiel 8: Extrakt von Guineapfeffer mit Kollagenhydrolysat und Chondroitin

3 kg trockene Guinea-Pfefferkörner (Aframomum melegueta) wurden zweimal mit je 10 Litern Ethanol bei 50°C für die Dauer von 8h extrahiert. Die Auszüge wurden über die Droge und einen Schichtenfilter abfiltriert, vereinigt und auf einen Trockensubstanzanteil von ca. 20% schonend im Vakuum beigedampft. Die native Extraktausbeute betrug 10%.

Eine Mischung von 80% Polyvinylpyrrolidon (Kollidon 25) und 20% Kollagenhydrolysat (Gelita Sol LDA) wurden auf ebenfalls 20% Trockensubstanzanteil in Ethanol 50% gelöst.

Anschließend wurden beide Lösungen im Mengenverhältnis 1:3 eingewogen und unter ständigem Rühren portionsweise miteinander homogenisert. Das so entstehende Pfeffer-Copräzipitat wurde im Vakuum vom Lösemittel befreit und zu einer Trockenextraktzubereitung getrocknet. 100 mg einer solchen Zubereitung werden mit 200 mg Glucosamin gemischt und über eine Zwangsvermahlung via 0,5 mm Sieb zu einem homogenen, frei fließenden Pulver verarbeitet.

### Vergleichsbeispiel 9: Extrakt von Salbeiblätern mit Kollagenhydrolysat

1 kg getrocknete und geschnittene Salbeiblätter (Salvia officinalis) wurden dreimal mit je 5 Litern EtOH 70% V/V bei 50°C für die Dauer von 4 h extrahiert. Die Auszüge wurden über die Droge abfiltriert, vereinigt und final über einen Schichtenfilter klarfiltriert. Anschließend wurde zum Dickextrakt lösemittelfrei im Vakuum beigedampft, wobei der aetherische Ölanteil separat aufgefangen wurde.

Anschließend wurde die wässrige Extraktlösung einer Membranfiltration unterzogen.

Der hiernach gewonnene Dickextrakt ergab eine native Extraktausbeute von 31%.

Diese Extraktlösung wurde auf einen TS von 35% im Vakuum eingeengt und mit 40% Kollagenhydrolysat (Gelita Sol LDA) unter Rühren homogenisiert.

In diesem Homogenisierungsprozess wurde auch das zuvor abgetrennte aetherische Öl wieder beigegeben. Die gesamte Lösung wurde sprühgetrocknet.

Es resultierte ein braun-beiges Trockenpulver. Der Extrakt enthielt 0,2% aetherisches Öl, ca. 5% Polyphenole (UV-VIS) und war komplett wasserlöslich.

### Beispiel 10: Kautablette

Die empfohlene Tagesdosierung entspricht 5 g Drogenpulver pro Tag. Dies entspricht beim DEV der Extraktzubereitung gemäß Beispiel 4a von 1:1 ebenfalls einer Dosierung von 5 g. Da die klassische Form von Tabletten oder Kapseln zum Schlucken bei einer Anzahl von ca. 6 Stück pro Tag zu Lasten der Compliance geht, werden Darreichungen für mehrere Gramm bevorzugt. Eine geschmacklich bevorzugte Variante ist eine aromatisierte Kautablette. Gemäß nachfolgender Rezeptur beträgt die empfohlene Tagesdosis 4 Stück.

**1 Kautablette (2 g) enthält:**

| | |
|---|---|
| Extraktzubereitung nach Beispiel 4a | 1,5 g |
| Sorbit | 0,3 g |
| PEG 4000 | 0,15 g |
| Aroma | 0,03 g |
| Calciumbehenat | 0,02 g. |

### Beispiel 11: Kaugummis

100 g Chicle werden gepulvert, mit 250 g Zuckeraustauschstoff Isomalt vermischt und in einer Abdampfschale erhitzt, bis die Masse weich wird. Sie wird dann gut unter Zugabe von 66 g Cynosbati-Trockenextrakt (gemäß Beispiel 4b) und 33 gr Calciumpyruvat durchgearbeitet und auf eine mit Stärke bestreute Platte gebracht und bis zur Gleichmäßigkeit geknetet. Eine zusätzliche Aromatisierung kann im vorangegangenen Schritt ebenso erfolgen. Schließlich wird sie in dünne Blätter ausgerollt und noch warm in flache Stangen geschnitten, indem man durch etwas Stärkepulver das Ankleben der Masse an der Platte verhütet. Die Kaugummi-Portionen sollen 2 Gramm betragen und die Portion enthält ca. 300 mg des Cynosbati-Extraktes.

### Beispiel 12: Brausegranulat oder -tablette

Zur Herstellung der Brausetabletten werden 600 g Zitronensäure mit 300 g Natriumhydrogencarbonat und 100 g des Cynosbati-Extraktes (gemäß Beispiel 4a) und 100 g Calciumpyruvat-Monohydrat gemischt. Dieser Mischung werden 50 g Mannit, 25 g eines Fruchtaromas, 5 g Saccharin und 20 g Natriumcyclamat zugesetzt. Nach erfolgter Homogenisierung kann das Gemisch granuliert oder direkt zu Tabletten verpresst werden. Als Einzeldosierung werden 5 g Granulat oder eine 5 g-Brausetablette 3-4 mal täglich empfohlen.

### Beispiel 13: Trinkfertige Darreichungen

Aufgrund der guten Löslichkeit des Extraktpulvers kann jedoch auch eine fertige Trinkzubereitung in Form von Einzeldosisampullen oder Fluida bzw. Sirups mit Dosierlöffel leicht dargestellt werden. Für derartige flüssige Mischungen empfiehlt sich eine Tagesdosis von 1,5 g des Extraktes nach Beispiel 4b. Neben geeigneten Geschmackszusätzen sind Zusätze von Ca-Supplementen oder löslichen Kollagenhydrolysaten vom Typ 1 als Kombinationspartner besonders geeignet.

## Patentansprüche

1. Zusammensetzung enthaltend einen entzündungshemmenden Pflanzenextrakt aus Hagebutte zusammen mit Kollagenhydrolysaten.

2. Zusammensetzung nach Anspruch 1, wobei der Pflanzenextrakt aus Hagebutte erhältlich ist durch ein Verfahren mit folgenden Schritten
a) Extraktion von Hagebuttenschalen mit Wasser oder einer Mischung von Wasser und bis zu 50 Gew.-% Ethanol um einen Einfachextrakt zu erhalten
b) Aufreinigung des erhaltenen Einfachextraktes durch mindestens einen der Schritte
b1) enzymatische Fermentierung
b2) Membranfiltration
c) Trocknung des Extraktes.

3. Verfahren zur Herstellung einer Zusammensetzung nach Anspruch 1 oder 2 mit folgenden Schritten:
a) Extraktion von Hagebuttenschalen mit Wasser oder einer Mischung von Wasser und bis zu 50 Gew.-% Ethanol um einen Einfachextrakt zu erhalten
b) Aufreinigung des erhaltenen Einfachextraktes durch mindestens einen der Schritte
b1) enzymatische Fermentierung mit hydrolytischen Enzymen
b2) Membranfiltration
c) Trocknung des Extraktes, wobei die Trocknung zusammen mit Kollagenhydrolysat erfolgt.

4. Zusammensetzung nach mindestens einem der Ansprüche 1 oder 2 zur Verwendung zur Prävention und zur Reduzierung von Symptomen bei Gelen kbeschwerden.

5. Zusammensetzung zur Verwendung nach Anspruch 4 zur Prävention oder Reduzierung von Symptomen bei chronischen Gelenkentzündungen, rheumatoider Arthritis, Erkrankungen des rheumatischen Formkreises, Spondylitis (speziell der S. ankylosans), Osteoarthritis, Arthrose, Fibromyalgie oder zur Unterstützung der Rehabilitation nach Gelenkverstauchung oder Bandscheibenstauchungen.

6. Arzneimittel enthaltend eine Zusammensetzung nach mindestens einem der Ansprüche 1 oder 2 in Form einer Tablette, einer Kautablette, eine Hartgelatinekapsel, einer Weichgelatinekapsel, einer Pastille, eines Sticks, eines Sachets oder in Form von flüssigen Darreichungsformen wie Einzeldosisampullen, Fluida und Sirupen.

7. Nahrungsergänzungsmittel enthaltend" eine Zusammensetzung nach mindestens einem der Ansprüche 1 oder 2 in Form einer Tablette, einer Kautablette, eine Hartgelatinekapsel, einer Weichgelatinekapsel, einer Pastille, eines Sticks, eines Sachets oder in Form von flüssigen Darreichungsformen wie Einzeldosisampullen, Fluida und Sirupen.

8. Bilanzierte Diät umfassend eine Zusammensetzung nach mindestens einem der Ansprüche 1 oder 2 in Form einer Tablette, einer Kautablette, eine Hartgelatinekapsel, einer Weichgelatinekapsel, einer Pastille, eines Sticks, eines Sachets oder in Form von flüssigen Darreichungsformen wie Einzeldosisampullen, Fluida und Sirupen.

## Claims

1. A composition comprising an anti-inflammatory plant extract from rose hips together with collagen hydrolysates.

2. The composition according to claim 1, wherein said plant extract from rose hips is obtainable by a process comprising the following steps:
a) extracting rose hip peel with water or a mixture of water and up to 50% by weight ethanol to obtain a single extract;
b) purifying the single extract obtained by at least one of the steps:
b1) enzymatic fermentation;
b2) membrane filtration
c) drying the extract.

3. A process for preparing a composition according to claim 1 or 2, comprising the following steps:
a) extracting rose hip peel with water or a mixture of water and up to 50% by weight ethanol to obtain a single extract;
b) purifying the single extract obtained by at least one of the steps:
b1) enzymatic fermentation with hydrolytic enzymes;
b2) membrane filtration
c) drying the extract, wherein said drying is effected together with collagen hydrolysate.

4. The composition according to at least one of claims 1 or 2 for use for the prevention and reduction of symptoms in joint complaints.

5. The composition for use according to claim 4 for the prevention or reduction of symptoms in chronic joint inflammations, rheumatoid arthritis, diseases of the rheumatic spectrum, spondylitis (especially S. ankylosans), osteoarthritis, arthrosis, fibromyalgia, or for supporting the rehabilitation after joint sprain or intervertebral disk compressions.

6. A medicament comprising a composition according to at least one of claims 1 or 2 in the form of a tablet, chewing tablet, hard gelatin capsule, soft gelatin capsule, lozenge, stick, sachet or in the form of liquid dosage forms, such as single-dose ampoules, fluids and syrups.

7. A food supplement comprising a composition according to at least one of claims 1 or 2 in the form of a tablet, chewing tablet, hard gelatin capsule, soft gelatin capsule, lozenge, stick, sachet or in the form of liquid dosage forms, such as single-dose ampoules, fluids and syrups.

8. A balanced diet comprising a composition according to at least one of claims 1 or 2 in the form of a tablet, chewing tablet, hard gelatin capsule, soft gelatin capsule, lozenge, stick, sachet or in the form of liquid dosage forms, such as single-dose ampoules, fluids and syrups.

## Revendications

1. Composition comprenant un extrait de plantes anti-inflammatoire constitué de cynorhodon conjointement avec des hydrolysats de collagène.

2. Composition selon la revendication 1, l'extrait de plantes constitué de cynorhodon pouvant être obtenu au moyen d'un procédé comprenant les étapes suivantes .
a) extraction d'écorces de cynorhodon avec de l'eau ou avec un mélange d'eau et jusqu'à 50 % en poids d'éthanol en vue d'obtenir un extrait simple
b) purification de l'extrait simple obtenu par au moins l'une des étapes suivantes :
b1) fermentation enzymatique
b2) filtration sur membrane
c) séchage de l'extrait.

3. Procédé de fabrication d'une composition selon la revendication 1 ou 2, comprenant les étapes suivantes :
a) extraction d'écorces de cynorhodon avec de l'eau ou un mélange d'eau et jusqu'à 50 % en poids d'éthanol en vue d'obtenir un extrait simple
b) purification de l'extrait simple obtenu par au moins l'une des étapes suivantes :
b1) fermentation enzymatique avec des enzymes hydrolytiques
b2) filtration sur membrane
c) séchage de l'extrait, le séchage ayant lieu conjointement avec de l'hydrolysat de collagène.

4. Composition selon au moins l'une des revendications 1 ou 2 destinée à être utilisée dans la prévention et la réduction de symptômes liés aux troubles articulaires.

5. Composition destinée à être utilisée selon la revendication 4 pour la prévention ou la réduction de symptômes liés aux inflammations articulaires chroniques, à la polyarthrite rhumatoïde, aux maladies rhumatismales, à la spondylarthrite (en particulier la spondylarthrite ankylosante), à l'ostéoarthrite, l'arthrose, la fibromyalgie ou pour aider à la rééducation après une entorse des articulations ou une compression des disques intervertébraux.

6. Médicament comprenant une composition selon au moins l'une des revendications 1 ou 2 sous la forme d'un comprimé, d'un comprimé à croquer, d'une gélule de gélatine dure, d'une gélule de gélatine molle, d'une pastille, d'un stick, d'un sachet ou de formes d'administration liquide, telles que des ampoules à dose unique, des fluides et des sirops.

7. Compléments alimentaires comprenant une composition selon au moins l'une des revendications 1 ou 2 sous forme d'un comprimé, d'un comprimé à croquer, d'une gélule de gélatine dure, d'une gélule de gélatine molle, d'une pastille, d'un stick, d'un sachet ou de formes d'administration liquide, telles que des ampoules à dose unique, des fluides et des sirops.

8. Aliment diététique destiné à des fins médicales spéciales, comprenant une composition selon au moins l'une des revendications 1 ou 2 sous forme d'un comprimé, d'un comprimé à croquer, d'une gélule de gélatine dure, d'une gélule de gélatine molle, d'une pastille, d'un stick, d'un sachet ou de formes d'administration liquide, telles que des ampoules à dose unique, des fluides et des sirops.
